# EUROPEAN PATENT SPECIFICATION

(11) **EP 4 326 357 B1**
(45) Date of publication and mention of the grant of the patent: **18.03.2026**
(21) Application number: 22724043.9
(22) Date of filing: 20.04.2022
(51) Int. Cl.: A61M 1/00, A61M 3/02

(54) **INTELLIGENT DISPOSABLE DEVICES FOR WOUND THERAPY AND TREATMENT**
INTELLIGENTE EINWEGVORRICHTUNGEN ZUR WUNDTHERAPIE UND -BEHANDLUNG
DISPOSITIFS JETABLES INTELLIGENTS POUR LA THÉRAPIE ET LE TRAITEMENT DE PLAIES

(30) Priority: 21.04.2021 GB 202105665
(43) Date of publication of application: 28.02.2024
(73) Proprietor: T.J. Smith and Nephew, Limited, Hull HU3 2BN (GB)
(72) Inventor: BREARLEY, David Joseph, Hull HU3 2BN (GB); GREGORY, Robert, Hull HU3 2BN (GB); HARTWELL, Edward Yerbury, Hull HU3 2BN (GB); HUNT, Allan Kenneth Frazer Grugeon, Hull HU3 2BN (GB); MILNER, Christopher James, Hull HU3 2BN (GB); QUINTANAR, Felix Clarence, Hull HU3 2BN (GB); STRACHAN, Kirsty Margaret, Hull HU3 2BN (GB)
(74) Representative: Appleyard Lees IP LLP
(86) International application number: PCT/EP2022/060459
(87) International publication number: WO 2022/223643

(56) References cited:
- US-A1- 2019 282 738
- US-A1- 2020 139 024

## Description

### Technical Field

Embodiments described herein relate to the use of intelligent disposable or consumable accessories with apparatuses, systems, and methods for the treatment of wounds, for example with negative pressure wound therapy or liquid jet debridement and wound bed preparation systems.

### Description of the Related Art

Traditional consumable or disposable accessories used in connection with negative pressure wound therapy or liquid jet debridement and wound bed preparation devices and systems lack intelligence. These traditional disposables function when they are connected and interfaced with devices and systems. However, no information related to the one or more properties the disposables is recorded and subsequently used to adjust provision of therapy or treatment. Document US2020139024 is considered as part of the prior art.

### SUMMARY

A canister for negative pressure wound therapy can include a housing defining an interior volume for storing fluid aspirated from a wound during provision of negative pressure wound therapy. The canister can include an electronic circuitry at least partially enclosed by the housing. The electronic circuitry can include a memory configured to store data related to one or more parameters of the canister. The electronic circuitry can include a transceiver configured to wirelessly communicate with a computing device. The electronic circuitry can include a processing circuitry connected to the memory and transceiver. The processing circuitry can be configured to store the data in the memory and cause the transceiver to provide at least some of the data stored in the memory responsive to a request from the computing device.

The negative canister of any of the preceding paragraphs and/or any of the canisters disclosed herein can include one or more of the following features. The memory can be configured to store code for updating firmware or software of the computing device, which can be a negative pressure wound therapy device configured to removably support the housing. The memory can be configured to store an indication for the negative pressure wound therapy device to determine that firmware or software should be updated with the code. The indication can include a version of firmware of software stored in the memory and configured to be executed by the processing circuitry. The transceiver can be configured to communicate with the computing device using a wireless communication protocol, such as near field communication (NFC). The computing device can be a negative pressure wound therapy device configured to removably support the housing. The data can include canister capacity stored in the memory prior to the canister being used for negative pressure wound therapy. The data can include instructions for recycling the canister. The data can include an indication of reaching end of life of the canister. The data can include an indication of the canister being used for providing negative pressure to a patient. The data can include canister orientation or canister fill status determined during provision of negative pressure wound therapy and stored in the memory during provision of negative pressure wound therapy. The canister orientation can include a flipped orientation determined during provision of negative pressure wound therapy. Storing the flipped orientation in the memory can prevent the canister from being used for further provision of negative pressure wound therapy. The electronic circuitry can include a power source configured to supply power to at least one of the memory, the transceiver, or the processing circuitry. The power source can include one or more of a capacitor or a battery. The data can include a manufacturing date stored as read only data. The manufacturing date can facilitate a determination that shelf life of the canister has not been exceeded. The data can include an indication that the canister is full. The indication can be stored responsive to a determination that a level of fluid aspirated from the wound has reached a threshold level over a duration of time.

A handpiece for a wound debridement system can include a housing configured to be coupled to a console and apply liquid jet to a wound. The handpiece can include an electronic circuitry at least partially enclosed by the housing. The electronic circuitry can include a memory configured to store data related to one or more parameters of the handpiece. The electronic circuitry can include a transceiver configured to communicate with the console. The electronic circuitry can include a processing circuitry connected to the memory and transceiver. The processing circuitry can be configured to store the data in the memory and cause the transceiver to provide at least some of the data stored in the memory responsive to a request from the console. The handpiece can include a mechanism configured to provide an indication that the handpiece has lost sterility.

The handpiece of any of the preceding paragraphs and/or any of the handpieces disclosed herein can include one or more of the following features. The mechanism can include a material configured to become nonconductive responsive to being exposed to the atmosphere for a duration of time. The mechanism can include a photodiode coupled to a material configured to change an optical property responsive to being exposed to the atmosphere for a duration of time. The data can include a liquid pressure value or range that the handpiece is capable of providing. Data related to the liquid pressure value or range can be stored in memory prior to use of the handpiece for debriding the wound. The console can be configured to alert a user to a liquid pressure outside of the liquid pressure value or range. The data can include instructions for recycling the handpiece. The data can include an indication of reaching end of life of the handpiece. The data can include an indication of the handpiece being used for debriding a wound of a patient.

A disposable device for use with a wound treatment or monitoring system can include a substrate or a hosing. The device can include an electronic circuitry at least partially supported by the substrate or housing. The electronic circuitry can include a memory configured to store data related to one or more parameters of the disposable device. The electronic circuitry can include a transceiver configured to wirelessly communicate with a computing device. The electronic circuitry can include a processing circuitry connected to the memory and transceiver. The processing circuitry can be configured to store the data in the memory and cause the transceiver to provide at least some of the data stored in the memory responsive to a request from the computing device.

The disposable device of any of the preceding paragraphs and/or any of the disposable devices disclosed herein can include one or more of the following features. The disposable device can include a mechanism configured to provide an indication that the disposable device has lost sterility. The mechanism can include a material configured to become nonconductive responsive to being exposed to the atmosphere for a first duration of time or a photodiode coupled to a material configured to change an optical property responsive to being exposed to the atmosphere for a second duration of time. The transceiver can be configured to communicate with the computing device using a wireless communication protocol, such as near field communication (NFC) or radio frequency identification (RFID). At least some of the data can be stored in the memory prior to the device being used for wound treatment or monitoring. At least some of the data can be stored in the memory responsive to the device being used for wound treatment or monitoring. The data can include an indication of whether the disposable device has been used for wound treatment or monitoring. The data can include instructions for recycling the device. The data can include an indication of reaching end of life of the device. The electronic circuitry can include a power source configured to supply power to at least one of the memory, the transceiver, or the processing circuitry. The power source can be one or more of a capacitor or a battery. The disposable device can be a sensor-integrated substrate, a wound dressing configured to provide vibrational therapy, or a wound dressing supporting a source of negative pressure.

A method (which is not part of the invention; this last one is solely defined by the appended set of claims) of using a disposable device of any of the preceding paragraphs and/or any of the disposable devices disclosed herein can include transferring data stored in a memory of the disposable device to a computing device and displaying the data on the computing device.

The method of the preceding paragraph and/or any of the methods disclosed herein can include one or more of the following features. At least some of the data can be stored in the memory prior to the disposable device being used for wound treatment or monitoring. At least some of the data can be stored in the memory responsive to the disposable device being used for wound treatment or monitoring.

Disclosed herein are methods of using and/or operating a canister, wound debridement system, or disposable device of any of the preceding paragraphs and/or any of the devices, canisters, apparatuses, or systems disclosed herein.

Disclosed herein are kits including the canister of any of the preceding paragraphs and/or any of the canisters disclosed herein and a negative pressure wound therapy device.

Disclosed herein are kits that include the canister of negative pressure wound therapy device of any of the preceding paragraphs and/or any of the devices, apparatuses, or systems disclosed herein and one or more wound dressings.

Any of the features, components, or details of any of the arrangements or embodiments disclosed in this application, including without limitation any of the apparatus embodiments and any of the negative pressure wound therapy embodiments disclosed herein, are interchangeably combinable with any other features, components, or details of any of the arrangements or embodiments disclosed herein to form new arrangements and embodiments.

### BRIEF DESCRIPTION OF THE DRAWINGS

Figure 1A illustrates a negative pressure wound therapy system.
Figure 1B illustrates another negative pressure wound therapy system.
Figure 2A is an isometric view of a negative pressure wound therapy device and canister, showing the canister detached from the pump assembly of the device.
Figure 2B is a back view of the negative pressure wound therapy device shown in Figure 2A.
Figure 2C illustrates a top surface of the negative pressure wound therapy device shown in Figure 2A, showing a user interface.
Figure 3 illustrates a schematic of a control system of a negative pressure wound therapy device.
Figure 4A illustrates a perspective view of a substrate supporting electronic components.
Figures 4B-4C illustrate perspective and top views of a perforated substrate supporting electronic components.
Figure 5 illustrates a liquid jet debridement and wound bed preparation system.
Figure 6A-6B illustrate a console of a liquid jet debridement and wound bed preparation system.
Figure 7 illustrates a schematic of an intelligent disposable.

### DETAILED DESCRIPTION

### Overview

An intelligent or smart disposable for use with a wound therapy or treatment system can advantageously store status data that facilitates more effective and efficient operation of the system. Status data can be stored in a memory of a smart disposable and can be accessed by another component of the system. Status data can be used to manage the life cycle of the smart disposable or the system, such as, set-up, adjusting the operation, or disposal of the smart disposable or the system. As described herein, wound therapy or treatment can be improved.

Smart disposables can be used for a variety of applications including, but not limited, to negative pressure wound therapy devices, wound debridement devices, wearable devices, wellness devices, therapy applicators, radiation applicators, measurement assembly devices, implantable devices, or semi-implantable devices. The smart disposable can be an integral part of a device and system when it is connected, activated, and identified. The smart disposable can communicate status data via wired or wireless communication protocol, such as radio frequency identification (RFID), near-field protocol (such as, near field communication (NFC)), infrared (such as, IrDA), vibration, or the like.

Generally, a smart disposable can allow access to the status data before or after the disposable has been used for provision of therapy or wound treatment. Status data can include, for example, the who, what, where, when, and how the disposable was or being used. Status data can be used to adjust therapy, preset alarms and alerts, control basic safety with premeasured data (for example, part temperature, electrical insolation, operating temperature, and the like), measure data to be used as a reference or compensation, measure radio frequency (RF) antenna parameters, individual data of RF wireless power and sensitivity, or measure impedance, capacitance, inductance, or temperature. Status data can include pre-calibration parameters, such as maximum pressure levels, vacuum source levels, or leak levels. The smart disposable can track shelf life status, time of manufacture, location or country of manufacture, shipping conditions, storage location, duration of use, or the like.

Status data can be used to adjust system level regulatory behavior. For example, one or more of the following can be adjusted: therapy parameters, safety and electromagnetic compatibility (EMC) deviations, waste management regulation data, biohazard regulation data, and location and privacy regulations. Status data can include system level functional use data, such as, data related to any failures, alarms and alerts, misuse, extreme operating use, and warranty breaks or failures. Status data can include an indication of the disposable having been used, which can be used to ensure and enforce single patient use while also allowing for reuse after an approved sterilization process. Status data can include instructions for use or instructions for disposal of one or more of the smart disposable or the wound therapy or treatment system.

The smart disposable may include one or more processors, memories, sensors, or communication interfaces. The smart disposable may be powered remotely or have a power source. Embodiments of a smart disposable may function as an accessory. The smart disposable may be able to communicate with other devices, systems, trackers, beacons, mesh networks, locators, waste processing apparatuses, software applications, smart phones, tablets, personal computers (PCs), or the like. The communication to and from the disposable may be established via wires and connectors, wirelessly, acoustically, optically, fluidically, using mechanical vibrations, pressure variations, and the like. The incorporation of a smart disposable can create an effective integral system capable of fully controlling the disposable's life cycle. The use of a smart disposable that stores status data can be advantageous over the use of labels, quick response (QR) codes, barcodes, or the like as these may be removed or damaged.

### Treating and Monitoring a Wound

Embodiments disclosed herein relate to smart disposables and systems and methods of treating or monitoring that interface with the smart disposables. Some examples relate to negative pressure wound therapy systems that include a negative pressure source configured provide negative pressure, via a fluid flow path, to a wound covered by a wound dressing. Some examples relate to wound debridement systems used to prepare the wound. Some examples relate to sensor-integrated wound dressing. Some examples relate to wound dressings that can provide vibrational therapy (such as, ultrasound). Some examples relate to wound dressings that support a negative pressure source, such as those described in International Patent Publication No. WO2020/187641.

Throughout this specification reference is made to a wound. The term wound is to be broadly construed and encompasses open and closed wounds in which skin is torn, cut or punctured or where trauma causes a contusion, or any other superficial or other conditions or imperfections on the skin of a patient or otherwise that benefit from pressure treatment. A wound is thus broadly defined as any damaged region of tissue where fluid may or may not be produced. Examples of such wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, bums, diabetic ulcers, pressure ulcers, stoma, surgical wounds, trauma and venous ulcers or the like.

Embodiments of the devices, systems, and methods disclosed herein can be used with topical negative pressure ("TNP") or reduced pressure therapy systems. Briefly, negative pressure wound therapy assists in the closure and healing of many forms of "hard to heal" wounds by reducing tissue oedema, encouraging blood flow and granular tissue formation, or removing excess exudate and can reduce bacterial load (and thus infection risk). **In** addition, the therapy allows for less disturbance of a wound leading to more rapid healing. TNP therapy systems can also assist in the healing of surgically closed wounds by removing fluid. TNP therapy can help to stabilize the tissue in the apposed position of closure. A further beneficial use of TNP therapy can be found in grafts and flaps where removal of excess fluid is important and close proximity of the graft to tissue is required in order to ensure tissue viability.

As used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects pressure that is X mmHg below 760 mmHg or, in other words, a pressure of (760-X) mmHg. In addition, negative pressure that is "less" or "smaller" than X mmHg corresponds to pressure that is closer to atmospheric pressure (for example, -40 mmHg is less than -60 mmHg). Negative pressure that is "more" or "greater" than -X mmHg corresponds to pressure that is further from atmospheric pressure (for example, -80 mmHg is more than -60 mmHg). In some cases, local ambient atmospheric pressure is used as a reference point, and such local atmospheric pressure may not necessarily be, for example, 760 mmHg.

Systems and methods disclosed herein can be used with other types of treatment in addition to or instead of reduced pressure therapy, such as irrigation, ultrasound, heat or cold, neuro stimulation, or the like. In some cases, disclosed systems and methods can be used for wound monitoring without application of additional therapy. Systems and methods disclosed herein can be used in conjunction with a dressing, including with compression dressing, reduced pressure dressing, or the like.

A healthcare provider, such as a clinician, nurse, or the like, can provide a TNP prescription specifying, for example, the pressure level or time of application. However, the healing process is different for each patient and the prescription may affect the healing process in a way the clinician or healthcare provider did not expect at the time of devising the prescription. A healthcare provider may try to adjust the prescription as the wound heals (or does not heal), but such process may require various appointments that can be time consuming and repetitive. Embodiments disclosed herein provide systems, devices, or methods of efficiently adjusting TNP prescriptions and delivering effective TNP therapy.

### Negative Pressure Wound Therapy with Smart Disposable

Any embodiment of a wound therapy system or device described herein may be configured with a smart disposable to provide any of the benefits described herein.

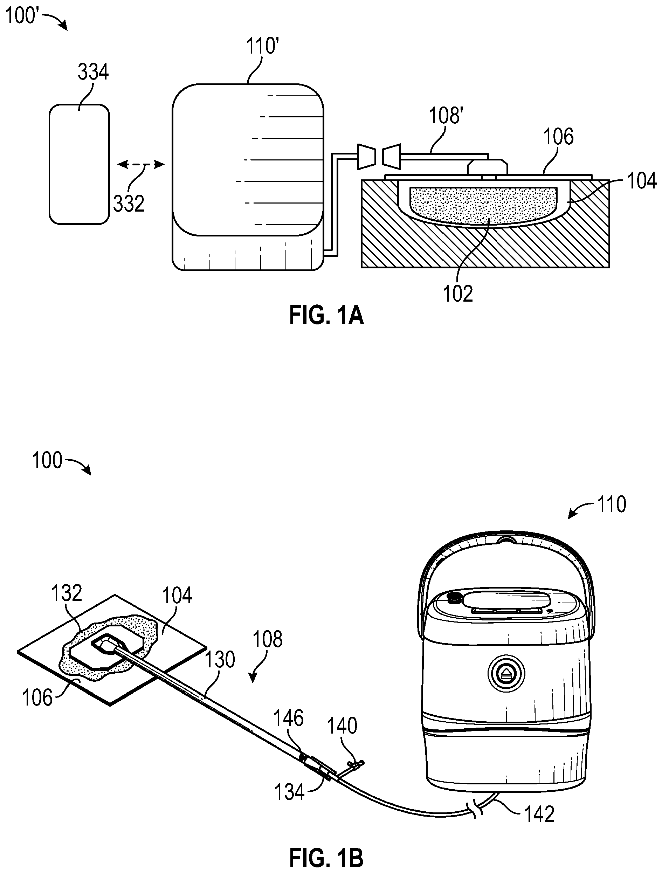

Figure 1A schematically illustrates a negative pressure wound treatment system 100' (sometimes referred to as a reduced or negative pressure wound therapy system, a TNP system, or a wound treatment system). In any implementations disclosed herein, though not required, the negative pressure wound treatment system 100' can include a wound filler 102 placed on or inside a wound 104 (which may be a cavity). The wound 104 can be sealed by a wound cover 106, which can be a drape, such that the wound cover 106 can be in fluidic communication with the wound 104. The wound filler 102 in combination with the wound cover 106 can be referred to as a wound dressing. A tube or conduit 108' (also referred to herein as a flexible suction adapter or a fluidic connector) can be used to connect the wound cover 106 with a wound therapy device 110' (sometimes as a whole or partially referred to as a "pump assembly") configured to supply reduced or negative pressure. The conduit 108' can be a single or multi lumen tube. With reference to Figure 1B, a connector 134 can be used to removably and selectively couple a conduit or tube 142 with the conduit 108'.

In any of the systems disclosed herein, a wound therapy device can be canisterless, wherein, for example and without limitation, wound exudate is collected in the wound dressing or is transferred via a conduit for collection at another location. However, any of the wound therapy devices disclosed herein can include or support a canister.

Additionally, with any of the wound therapy systems disclosed herein, any of the wound therapy devices can be mounted to or supported by the wound dressing or adjacent to the wound dressing. The wound filler 102 can be any suitable type, such as hydrophilic or hydrophobic foam, gauze, inflatable bag, and so on. The wound filler 102 can be conformable to the wound 104 such that the wound filler 102 substantially fills the cavity of the wound 104. The wound cover 106 can provide a substantially fluid impermeable seal over the wound 104. The wound cover 106 can have a top side and a bottom side. The bottom side can adhesively (or in any other suitable manner) seal with the wound 104, for example by sealing with the skin around the wound 104. The conduit 108' or any other conduit disclosed herein can be formed from polyurethane, PVC, nylon, polyethylene, silicone, or any other suitable material.

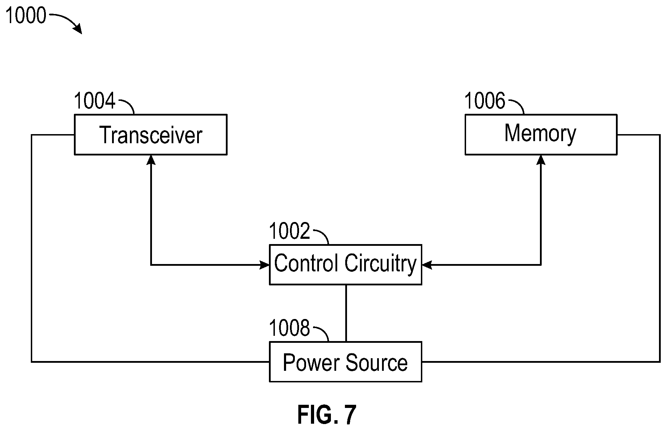

The wound cover 106 can have a port (not shown) configured to receive an end of the conduit 108'. In some cases, the conduit 108' can otherwise pass through or under the wound cover 106 to supply reduced pressure to the wound 104 so as to maintain a desired level of reduced pressure in the wound 104. The conduit 108' can be any suitable article configured to provide at least a substantially sealed fluid flow pathway or path between the wound therapy device 110' and the wound cover 106, so as to supply the reduced pressure provided by the wound therapy device 110' to wound 104. The pressure being provided may be stored as status data by a smart disposable, such as the smart disposable 1000 as shown in Figure 7.

The wound cover 106 and the wound filler 102 can be provided as a single article or an integrated single unit. In some cases, no wound filler is provided and the wound cover by itself may be considered the wound dressing. The wound dressing can then be connected, via the conduit 108', to a source of negative pressure of the wound therapy device 110'. In some cases, though not required, the wound therapy device 110' can be miniaturized and portable, although larger conventional negative pressure sources (or pumps) can also be used.

The wound cover 106 can be located over a wound site to be treated. The wound cover 106 can form a substantially sealed cavity or enclosure over the wound. The wound cover 106 can have a film having a high water vapour permeability to enable the evaporation of surplus fluid, and can have a superabsorbing material contained therein to safely absorb wound exudate. In some cases, the components of the TNP systems described herein can be particularly suited for incisional wounds that exude a small amount of wound exudate.

The wound therapy device 110' can operate with or without the use of an exudate canister. In some cases, as is illustrated, the wound therapy device 110' can include an exudate canister. In some cases, configuring the wound therapy device 110' and conduit 108' so that the conduit 108' can be quickly and easily removed from the wound therapy device 110' can facilitate or improve the process of wound dressing or pump changes, if necessary. Any of the pump assemblies disclosed herein can have any suitable connection between the conduit 108' and the pump. If an exudate canister is used with the wound therapy device 110' (or any of the other wound therapy devices disclosed herein), it can be configured as a smart disposable, such as the smart disposable 1000 as shown in Figure 7.

The wound therapy device 110' can deliver negative pressure of approximately -80 mmHg, or between about -20 mmHg and -200 mmHg. Note that these pressures are relative to normal ambient atmospheric pressure thus, -200 mmHg would be about 560 mmHg in practical terms. In some cases, the pressure range can be between about -40 mmHg and -150 mmHg. Alternatively, a pressure range of up to -75 mmHg, up to -80 mmHg or over -80 mmHg can be used. Also in some cases a pressure range of below -75 mmHg can be used. Alternatively, a pressure range of over approximately -100 mmHg, or even -150 mmHg, can be supplied by the wound therapy device 110'.

As will be described in greater detail below, the negative pressure wound treatment system 100' can be configured to provide a connection 332 to a separate or remote computing device 334. The connection 332 can be wired or wireless (such as, Bluetooth, Bluetooth low energy (BLE), NFC, WiFi, or cellular). The remote computing device 334 can be a smartphone, a tablet, a laptop or another standalone computer, a server (such as, a cloud server), another pump device, or the like. The remote computing device 334 can be incorporated with any wound therapy or treatment system disclosed herein and configured to communicate with a smart disposable as described herein.

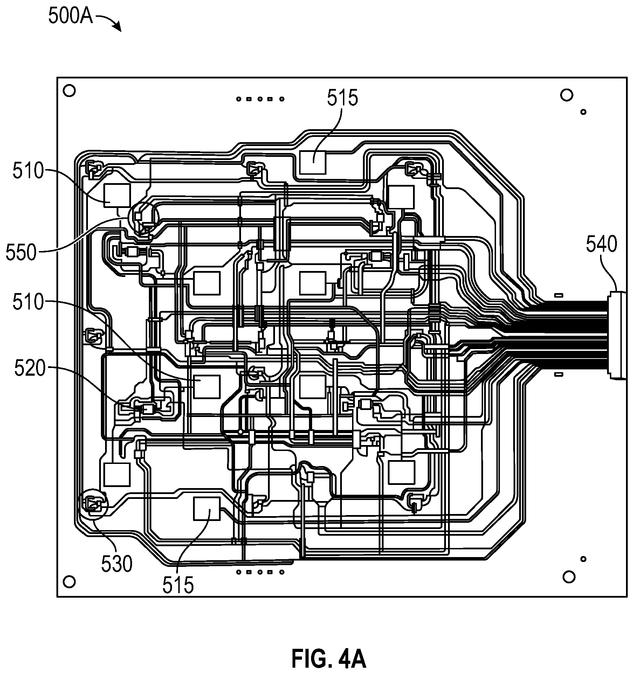

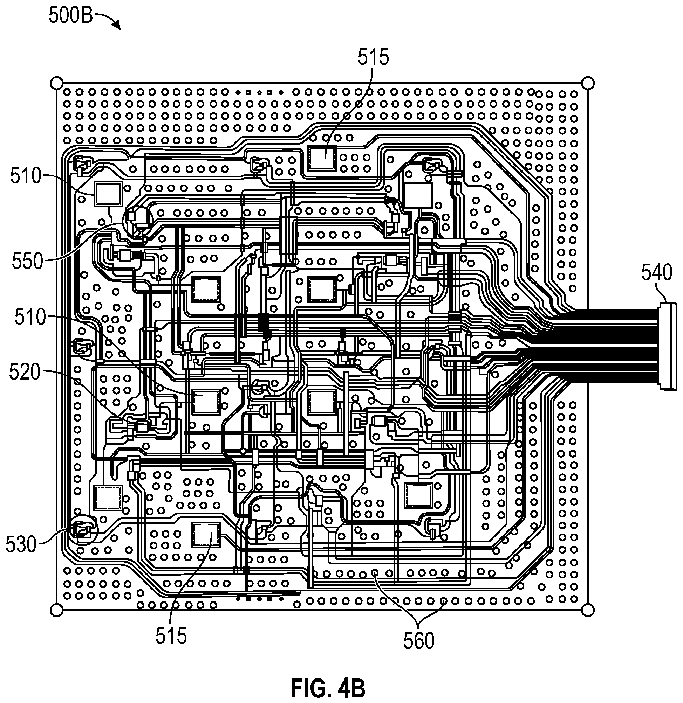

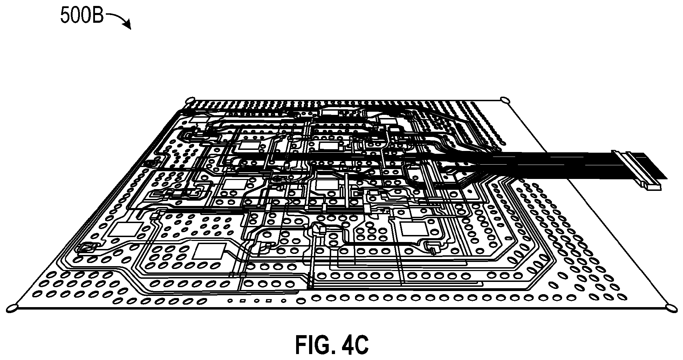

Figure 1B illustrates another negative pressure wound treatment system 100. The negative pressure wound treatment system 100 can have any of the components, features, or other details of any of the other negative pressure wound treatment system disclosed herein, including without limitation the negative pressure wound treatment system 100' illustrated in Figure 1A or the negative pressure wound treatment system 400 illustrated in Figure 4, in combination with or in place of any of the components, features, or other details of the negative pressure wound treatment system 100 shown in Figure 1B and/or described herein. The negative pressure wound treatment system 100 can have a wound cover 106 over a wound 104 that can seal the wound 104. A conduit 108, such as a single or multi lumen tube can be used to connect the wound cover 106 with a wound therapy device 110 (sometimes as a whole or partially referred to as a "pump assembly") configured to supply reduced or negative pressure. The wound cover 106 can be in fluidic communication with the wound 104.

With reference to Figure 1B, the conduit 108 can have a bridge portion 130 that can have a proximal end portion and a distal end portion (the distal end portion being closer to the wound 104 than the proximal end portion, and an applicator 132 at the distal end of the bridge portion 130 forming the flexible suction adapter (or conduit) 108. A connector 134 can be disposed at the proximal end of the bridge portion 130, so as to connect to at least one of the channels that can extend along a length of the bridge portion 130 of the conduit 108 shown in Figure 1B. A cap 140 can be coupled with a portion of the conduit 108 and can, in some cases, as illustrated, be attached to the connector 134. The cap 140 can be useful in preventing fluids from leaking out of the proximal end of the bridge portion 130. The conduit 108 can be a Soft Port manufactured by Smith & Nephew. As mentioned, the negative pressure wound treatment system 100 can include a source of negative pressure, such as the device 110, capable of supplying negative pressure to the wound 104 through the conduit 108. Though not required, the device 110 can also include a canister or other container for the storage of wound exudates and other fluids that can be removed from the wound.

The device 110 can be connected to the connector 134 via a conduit or tube 142. In use, the applicator 132 can be placed over an aperture formed in a cover 106 that is placed over a suitably-prepared wound or wound 104. Subsequently, with the wound therapy device 110 connected via the tube 142 to the connector 134, the wound therapy device 110 can be activated to supply negative pressure to the wound. Application of negative pressure can be applied until a desired level of healing of the wound is achieved.

The bridge portion 130 can comprise an upper channel material or layer positioned between an upper layer and an intermediate layer, with a lower channel material or layer positioned between the intermediate layer and a bottom layer. The upper, intermediate, and lower layers can have elongate portions extending between proximal and distal ends and can include a material that is fluid-impermeable, for example polymers such as polyurethane. It will of course be appreciated that the upper, intermediate, and lower layers can each be constructed from different materials, including semi-permeable materials. In some cases, one or more of the upper, intermediate, and lower layers can be at least partially transparent. In some instances, the upper and lower layers can be curved, rounded or outwardly convex over a majority of their lengths.

The upper and lower channel layers can be elongate layers extending from the proximal end to the distal end of the bridge 130 and can each preferably comprise a porous material, including for example open-celled foams such as polyethylene or polyurethane. In some cases, one or more of the upper and lower channel layers can be comprised of a fabric, for example a knitted or woven spacer fabric (such as a knitted polyester 3D fabric, Baltex 7970.RTM., or Gehring 879.RTM.) or a nonwoven material, or terry-woven or loop-pile materials. The fibers may not necessarily be woven, and can include felted and flocked (including materials such as Flotex.RTM.) fibrous materials. The materials selected are preferably suited to channeling wound exudate away from the wound and for transmitting negative pressure or vented air to the wound site, and can also confer a degree of kinking or occlusion resistance to the channel layers. In one example, the upper channel layer can include an open-celled foam such as polyurethane, and the lower channel layer can include a fabric. In another example, the upper channel layer is optional, and the system can instead be provided with an open upper channel. The upper channel layer can have a curved, rounded or upwardly convex upper surface and a substantially flat lower surface, and the lower channel layer can have a curved, rounded or downwardly convex lower surface and a substantially flat upper surface.

The fabric or material of any components of the bridge 130 can have a three-dimensional (3D) structure, where one or more types of fibers form a structure where the fibers extend in all three dimensions. Such a fabric can in some cases aid in wicking, transporting fluid or transmitting negative pressure. In some cases, the fabric or materials of the channels can include several layers of material stacked or layered over each other, which can in some cases be useful in preventing the channel from collapsing under the application of negative pressure. The materials used in some implementations of the conduit 108 can be conformable and pliable, which can, in some cases, help to avoid pressure ulcers and other complications which can result from a wound treatment system being pressed against the skin of a patient.

The distal ends of the upper, intermediate, and lower layers and the channel layers can be enlarged at their distal ends (to be placed over a wound site), and can form a "teardrop" or other enlarged shape. The distal ends of at least the upper, intermediate, and lower layers and the channel layers can also be provided with at least one through aperture. This aperture can be useful not only for the drainage of wound exudate and for applying negative pressure to the wound, but also during manufacturing of the device, as these apertures can be used to align these respective layers appropriately.

In some implementations, a controlled gas leak 146 (sometimes referred to as gas leak, air leak, or controlled air leak) can be disposed on the bridge portion 130, for example at the proximal end thereof. This air leak 146 can comprise an opening or channel extending through the upper layer of the bridge portion 130, such that the air leak 146 is in fluidic communication with the upper channel of the bridge portion 130. Upon the application of suction to the conduit 108, gas (such, as air) can enter through the gas leak 146 and move from the proximal end of the bridge portion 130 to the distal end of the bridge portion along the upper channel of the bridge portion 130. The gas can then be suctioned into the lower channel of the bridge portion 130 by passing through the apertures through the distal ends of the upper, intermediate, and lower layers. Data related to the air leak 146, such as the flow rate (or flow rate ranges) provided by the air leak 146, can be part of status data stored by a smart disposable, such as the smart disposable 1000 as shown in Figure 7.

The air leak 146 can include a filter. Preferably, the air leak 146 is located at the proximal end of the bridge portion 130 so as to minimize the likelihood of wound exudate or other fluids coming into contact and possibly occluding or interfering with the air leak 146 or the filter. In some instances, the filter can be a microporous membrane capable of excluding microorganisms and bacteria, and which may be able to filter out particles larger than 45 µm. Preferably, the filter can exclude particles larger than 1.0 µm, and more preferably, particles larger than 0.2 µm. Advantageously, some implementations can provide for a filter that is at least partially chemically-resistant, for example to water, common household liquids such as shampoos, and other surfactants. In some cases, reapplication of vacuum to the suction adapter or wiping of the exposed outer portion of the filter may be sufficient to clear any foreign substance occluding the filter. The filter can be composed of a suitably-resistant polymer such as acrylic, polyethersulfone, or polytetrafluoroethylene, and can be oleophobic or hydrophobic. In some cases, the gas leak 146 can supply a relatively constant gas flow that does not appreciably increase as additional negative pressure is applied to the conduit 108. In instances of the negative pressure wound treatment system 100 where the gas flow through the gas leak 146 increases as additional negative pressure is applied, preferably this increased gas flow will be minimized and not increase in proportion to the negative pressure applied thereto. Further description of such bridges, conduits, air leaks, and other components, features, and details that can be used with any implementations of the negative pressure wound treatment systems disclosed herein are found in U.S. Patent No. 8,801,685.

Any of the wound therapy devices (such as, the device 110 or 110') disclosed herein can provide continuous or intermittent negative pressure therapy. Continuous therapy can be delivered at above 0 mmHg, -25 mmHg, -40 mmHg, -50 mmHg, -60 mmHg, -70 mmHg, -80 mmHg, -90 mmHg, -100 mmHg, -120 mmHg, -125 mmHg, -140 mmHg, -160 mmHg, -180 mmHg, -200 mmHg, or below -200 mmHg. Intermittent therapy can be delivered between low and high negative pressure set points (sometimes referred to as setpoint). Low set point can be set at above 0 mmHg, -25 mmHg, -40 mmHg, -50 mmHg, -60 mmHg, -70 mmHg, -80 mmHg, -90 mmHg, -100 mmHg, -120 mmHg, -125 mmHg, -140 mmHg, -160 mmHg, -180 mmHg, or below -180 mmHg. High set point can be set at above -25 mmHg, -40 mmHg, -50 mmHg, -60 mmHg, -70 mmHg, -80 mmHg, -90 mmHg, -100 mmHg, -120 mmHg, -125 mmHg, -140 mmHg, -160 mmHg, -180 mmHg, -200 mmHg, or below -200 mmHg. During intermittent therapy, negative pressure at low set point can be delivered for a first time duration, and upon expiration of the first time duration, negative pressure at high set point can be delivered for a second time duration. Upon expiration of the second time duration, negative pressure at low set point can be delivered. The first and second time durations can be same or different values.

In operation, the wound filler 102 can be inserted into the cavity of the wound 104, and wound cover 106 can be placed so as to seal the wound 104. The wound therapy device 110' can provide negative pressure to the wound cover 106, which can be transmitted to the wound 104 via the wound filler 102. Fluid (such as, wound exudate) can be drawn through the conduit 108 and stored in a canister. In some cases, fluid is absorbed by the wound filler 102 or one or more absorbent layers (not shown).

Wound dressings that can be utilized with the pump assembly and systems of the present application include Renasys-F, Renasys-G, Renasys AB, and Pico Dressings available from Smith & Nephew. Further description of such wound dressings and other components of a negative pressure wound therapy system that can be used with the pump assembly and systems of the present application are found in U.S. Patent Publication Nos. 2012/0116334, 2011/0213287, 2011/0282309, 2012/0136325, U.S. Patent No. 9,084,845, and International App. No. PCT/EP2020/078376. In some cases, other suitable wound dressings can be utilized.

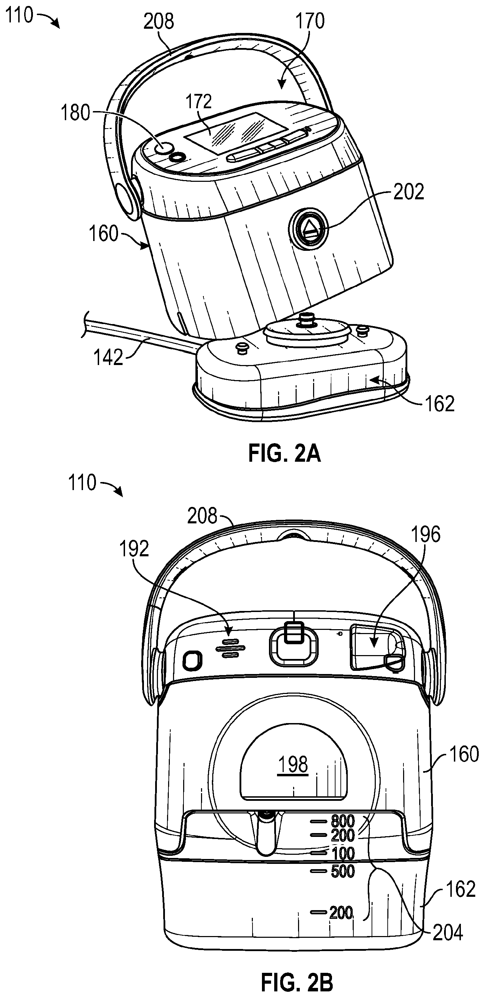

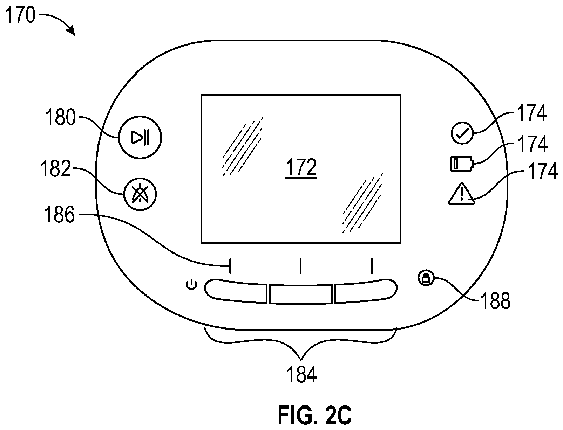

Figures 2A-2C show the negative pressure wound therapy device 110. As illustrated, a pump assembly 160 and canister 162 can be connected, thereby forming the wound therapy device 110. With reference to Figure 2C, the pump assembly 160 can include an interface panel 170 having a display 172, one or more indicators 174, or one or more controls or buttons, including, for example and without limitation, a therapy start and pause button 180 or an alarm/alert mute button 182. The interface panel 170 can have one or more input controls or buttons 184 (three being shown) that can be used to control any functions of the pump assembly 160 or the interface panel 170. For example and without limitation, one or more of the buttons 184 can be used to turn the pump assembly 160 on or off, to start or pause therapy, to operate and monitor the operation of the pump assembly 160, to scroll through menus displayed on the display 172, or to control or perform other functions. In some cases, the command buttons 184 can be programmable, and can be made from a tactile, soft rubber.

Additionally, the interface panel 170 can have visual indicators 186 that can indicate which of the one or more buttons 184 is active. The interface panel 170 can also have a lock/unlock control or button 188 that can be configured to selectively lock or unlock the functionality of the various buttons (e.g., buttons 184) or the display 172. For example, therapy setting adjustment can be locked/unlocked via the lock/unlock control 188. When the lock/unlock button 188 is in the locked state, depressing one or more of the various other buttons or the display will not cause the pump assembly 160 to change any display functions or performance functions of the device. This way, the interface panel 170 will be protected from inadvertent bumping or touching of the various buttons or display. The interface panel 170 can be located on an upper portion of the pump assembly 160, for example and without limitation on an upward facing surface of the pump assembly 160.

The display 172, which can be a screen such as an LCD screen, can be mounted in a middle portion of the interface panel 170. The display 172 can be a touch screen display. The display 172 can support playback of audiovisual (AV) content, such as instructional videos, and render a number of screens or graphical user interfaces (GUIs) for configuring, controlling, and monitoring the operation of the pump assembly 160.

The one or more indicators 174 can be lights (such as, LEDs) and can be configured to provide a visual indication of alarm conditions and or a status of the pump. For example and without limitation, the one or more indicators 174 can be configured to provide a visual indication of a status of the pump assembly 160 or other components of the negative pressure wound treatment system 100, including without limitation the conduit 108 or the wound cover 106 (such as, to provide an indication of normal operation, low battery, a leak, canister full, blockage, overpressure, or the like). Any one or more suitable indicators can be additionally or alternatively used, such as visual, audio, tactile indicator, and so on.

Figure 2B shows a back or rear view of the wound therapy device 110 shown in the Figure 2A. As shown, the pump assembly 160 can include a speaker 192 for producing sound. For example and without limitation, the speaker 192 can generate an acoustic alarm in response to deviations in therapy delivery, non-compliance with therapy delivery, or any other similar or suitable conditions or combinations thereof. Any of the alarms or alerts can be part of status data stored by a smart disposable. The speaker 192 can provide audio to accompany one or more instructional videos that can be displayed on the display 172.

The pump assembly 160 can be configured to provide easy access (such as, an access door on the casing of the pump assembly) to one or more filters of the pump assembly 160, such as antibacterial filters. This can enable a user (such as, a healthcare provider or patient) to more easily access, inspect or replace such filters. The pump assembly 160 can also include a power jack (or socket) 196 for providing power to the pump assembly 160 or for charging and recharging an internal power source (such as, a battery). Some implementations of the pump assembly 160 can include a disposable or renewable power source, such as one or more batteries, so that no power jack is needed. The pump assembly 160 can have a recess 198 formed therein to facilitate gripping of the pump assembly 160.

The canister 162 can hold fluid aspirated from the wound 104. For example, the canister 162 can have an 800 mL (or approximately 800 mL) capacity, or from a 300 mL or less capacity to a 1000 mL or more capacity, or any capacity level in this range. The canister 162 can include a tubing for connecting to the conduit 108 in order to form a fluid flow path. The canister 162 can be replaced with another canister, such as when the canister 162 has been filled with fluid. With reference to Figure 2A, the wound therapy device 110 can include a canister inlet tube 200 (also referred to herein as a dressing port connector) in fluid communication with the canister 162. For example and without limitation, the canister inlet tube 200 can be used to connect with the conduit 108.

The canister 162 can be selectively coupleable and removable from the pump assembly 160. With reference to Figure 2A, in some cases, a canister release button 202 can be configured to selectively release the canister 162 from the pump assembly 160. With reference to Figure 2B, the canister 162 can have one or more fill lines or graduations 204 to indicate to the user and amount of fluid or exudate stored within the canister 162.

The canister 162 can be configured as a smart disposable, such as the smart disposable 1000. For example, the canister 162 can include electronics with memory and processing capabilities. The electronics can be partially or fully positioned within the canister housing. The electronics can be powered by a power source, such as a coin cell battery or one or more capacitors. In some cases, external power can be provided, such as via NFC, RFID, or other wireless charging protocols. The canister 162 can facilitate system level activation and deactivation subsequent to the canister 162 having been connected to the pump assembly 160. As described herein, the canister 162 can store status data. Status data can include the canister 162 identifier (which can be unique), type, or capacity (such as, 350 mL or a 700 mL canister). This data can be used by the pump assembly 160 to adjust provision of negative pressure wound therapy.

In some instances, the duration of time for establishing a negative pressure set point can be adjusted based on the capacity (or size) of the canister. For a larger canister, amount of flow can be increased (for instance, by increasing the speed or a pump motor) to faster aspirate gas from the canister and establish the negative pressure set point. One or more of charging or performance can be adjusted based on the canister capacity. For instance, the user may expect that a larger canister would allow the user to be ambulatory for a longer duration. The device can respond by one or more of charging the power supply faster or execute one or more power saving techniques (such as, turn of the display). Alarms or alert delays and behavior can change based on the canister capacity. For instance, canister fill or full thresholds can be adjusted responsive to the detected canister capacity. As another example, the device may operate in vertical orientation with a smaller canister and in horizontal orientation with a larger canister (or vice versa). Alarms or alerts can be adjusted based on the position of the device during provision of therapy. In cases when irrigation can be provided along with the negative pressure, the amount or volume of instillation fluid provided can be adjusted based on the capacity of the canister. For instance, smaller volume of the fluid can be provided with a smaller canister.

An indication can be provided to the user responsive to detecting the capacity of the canister. For example, an ambulatory patient can be made aware of mobility limitations with a larger canister. As another example, an indication that a larger canister is a better option when the user may be travelling can be provided. Indication of the set of proper accessories, such as carry bag, straps, bed clams, IV pole, or transit case, can be provided based on the detected canister capacity. Any of such accessories may also be a smart accessory.

Status data can be used to adjust one or more tests performed during manufacturing. For example, detection of a larger canister can cause adjustment of one or more thresholds for a drop (or shock) test to account for the additional weight of the larger canister. For a heavier system, the warranty or acceptance of a drop (or shock) requirement can be more delicate than for a system with a smaller canister.

Status data can include vacuum level detected during manufacturing or testing. This data can be used by the pump assembly during provision of therapy, for example, to verify that the canister 162 is intact (such as, not cracked or damaged). Such verification can be made by comparing pressure measurement(s) by one or more pressure sensors during provision of therapy to the vacuum level stored as part of status data.

Status data can include an indication that the canister 162 has been used during therapy. This can facilitate ensuring that the canister is not used for providing therapy to a different patient (for instance, without having been cleaned and sterilized). The indication of the canister having been used can be stored in the memory of the canister 162 responsive to, for instance, detecting connection of the canister 162 to the pump assembly 160 or activation of the pump assembly 160 to provide negative pressure. Status data can include an indication that the canister 162 was tilted or turned upside down (which may compromise a filter of the canister and render the canister 1620 unusable for provision of negative pressure wound therapy). The indication of the canister 162 having been tilted or turned upside down can be stored in the memory of the canister 162 responsive to, for example, detecting orientation change of the pump assembly 160 and the canister. Such detection can be performed using one or more motion sensors of the pump assembly 160 (such as, an accelerometer or gyroscope).

Status data can include an indication that the canister 162 has reached end of life as a result of being in use for a particular time duration indicative of its operational lifetime. For example, the pump assembly 160 can track the cumulative duration of negative pressure wound therapy provided with the canister 162. In response to detecting that that cumulative duration has reached the particular time duration, an indication that end of life of the canister has been reached can be stored as part of the status data of the canister. As another example, timestamps (as described herein) can be used for detection of end of life. Status data can include location, hospital, use or misuse condition, environmental use, exposure of the canister (such as, remote or ambulatory), transportation use, or the like.

The pump assembly 160 can retrieve the status data from the canister 162. The retrieval can be performed by or under control of one or more controllers of the pump assembly 160. The status data can be retrieved prior to initiating negative pressure wound therapy. The pump assembly 160 can retrieve the status data using any of the approaches described in co-pending International Patent Application No. (Atty. Docket SMNPH.654WO), titled "Communication Systems and Methods for Negative Pressure Wound Therapy Devices" and co-pending International Application No. (Atty. Docket SMNPH.672WO), titled "Canister Status Determination for Negative Pressure Wound Therapy Devices". For instance, the pump assembly 160 can retrieve the status data using a near-field protocol (such as, NFC). The pump assembly 160 can adjust therapy responsive to the status data. For example, the pump assembly 160 can disallow provision of therapy responsive to a determination that the status data indicates that the canister 162 had already been in use or had been tilted or turned upside down. Use or misuse data can be used to manage or resolve complaints, warranties, or costs for the canister 162 or any of the smart disposables disclosed herein.

The wound therapy device 110 can have a handle 208 that can be used to lift or carry the wound therapy device 110. The handle 208 can be coupled with the pump assembly 160 and can be rotatable relative to the wound therapy device 110 so that the handle can be rotated upward for lifting or carrying the wound therapy device 110 or the pump assembly 160, or rotated into a lower profile in a more compact position when the handle is not being used. In some cases, the handle 208 can be coupled with the pump assembly 160 in a fixed position. The handle 208 can be coupled with an upper portion of the pump assembly 160 or can be removable from the wound therapy device 110.

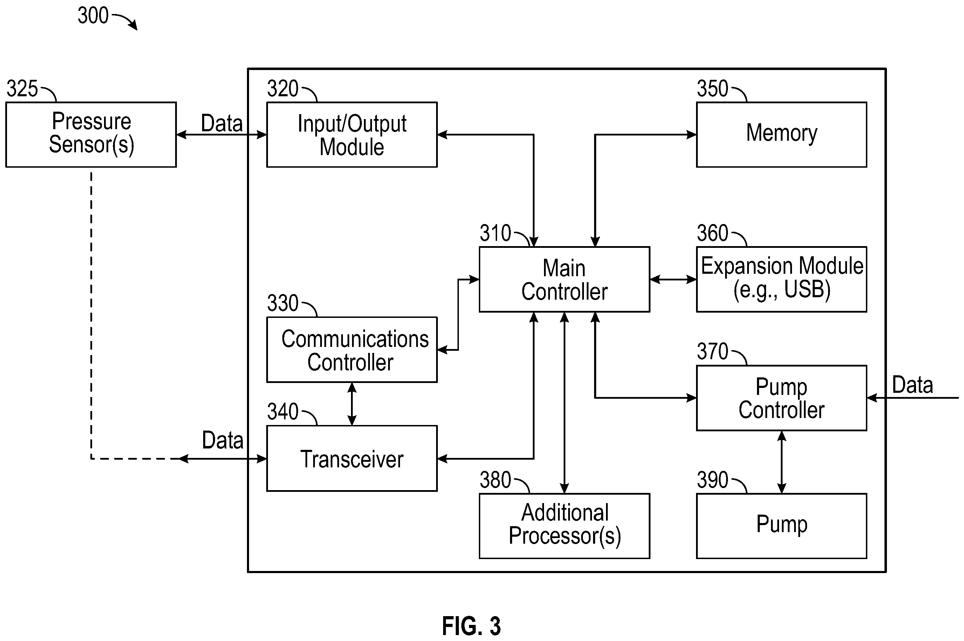

Figure 3 illustrates a schematic of a control system 300 that can be employed in any of the wound therapy devices described herein, such as in the wound therapy device 110' or 110. Electrical components can operate to accept user input, provide output to the user, operate the pressure source, provide connectivity, and so on. A first processor (such as, a main controller 310) can be responsible for user activity, and a second processor (such as, a pump controller 370) can be responsible for controlling another device, such as a pump 390.

An input/output (I/O) module 320 can be used to control an input and/or output to another component or device, such as the pump 390, one or more sensors (for example, one or more pressure sensors 325 configured to monitor pressure in one or more locations of the fluid flow path), or the like. For example, the I/O module can receive data from one or more sensors through one or more ports, such as serial (for example, I2C), parallel, hybrid ports, and the like. Any of the pressure sensors can be part of the wound therapy device or the canister. In some cases, any of the pressure sensors 325 can be remote to the wound therapy device, such as positioned at or near the wound (for example, in the dressing or the conduit connecting the dressing to the wound therapy device). In such implementations, any of the remote pressure sensors can communicate with the I/O module over a wired connection or with one or more transceivers 340 over a wireless connection.

The main controller 310 can receive data from and provide data to one or more expansion modules 360, such as one or more USB ports, SD ports, Compact Disc (CD) drives, DVD drives, FireWire ports, Thunderbolt ports, PCI Express ports, and the like. The main controller 310, along with other controllers or processors, can store data in memory 350 (such as one or more memory modules), which can be internal or external to the main controller 310. Any suitable type of memory can be used, including volatile or non-volatile memory, such as RAM, ROM, magnetic memory, solid-state memory, Magnetoresistive random-access memory (MRAM), and the like.

The main controller 310 can be a general purpose controller, such as a low-power processor or an application specific processor. The main controller 310 can be configured as a "central" processor in the electronic architecture of the control system 300, and the main controller 310 can coordinate the activity of other processors, such as the pump controller 370, one or more communications controllers 330, and one or more additional processors 380. The main controller 310 can run a suitable operating system, such as a Linux, Windows CE, VxWorks, etc.

The pump controller 370 can control the operation of a pump 390, which can generate negative or reduced pressure. The pump 390 can be a suitable pump, such as a diaphragm pump, peristaltic pump, rotary pump, rotary vane pump, scroll pump, screw pump, liquid ring pump, diaphragm pump operated by a piezoelectric transducer, voice coil pump, and the like. The pump controller 370 can measure pressure in a fluid flow path, using data received from one or more pressure sensors 325, calculate the rate of fluid flow, and control the pump. The pump controller 370 can control the pump actuator (such as, a motor) so that a desired level of negative pressure is achieved in the wound 104. The desired level of negative pressure can be pressure set or selected by the user. The pump controller 370 can control the pump (for example, pump motor) using pulse-width modulation (PWM) or pulsed control. A control signal for driving the pump can be a 0-100% duty cycle PWM signal. The pump controller 370 can perform flow rate calculations and detect alarms. The pump controller 370 can communicate information to the main controller 310. The pump controller 370 can be a low-power processor.

Any of the one or more communications controllers 330 can provide connectivity (such as, a wired or wireless connection 332). The one or more communications controllers 330 can utilize one or more transceivers 340 for sending and receiving data. The one or more transceivers 340 can include one or more antennas, optical sensors, optical transmitters, vibration motors or transducers, vibration sensors, acoustic sensors, ultrasound sensors, or the like. Any of the one or more transceivers 340 can function as a communications controller. In such case, the one or more communications controllers 330 can be omitted. Any of the one or more transceivers 340 can be connected to one or more antennas that facilitate wireless communication. The one or more communications controllers 330 can provide one or more of the following types of connections: Global Positioning System (GPS), cellular connectivity (for example, 2G, 3G, LTE, 4G, 5G, or the like), NFC, Bluetooth (or BLE) connectivity, radio frequency identification (RFID), wireless local area network (WLAN), wireless personal area network (WPAN), WiFi connectivity, Internet connectivity, optical connectivity (for example, using infrared light, barcodes, such as QR codes, etc.), acoustic connectivity, ultrasound connectivity, or the like. Connectivity can be used for various activities, such as pump assembly location tracking, asset tracking, compliance monitoring, remote selection, uploading of logs, alarms, and other operational data, and adjustment of therapy settings, upgrading of software or firmware, pairing, and the like.

Any of the one or more communications controllers 330 can provide dual GPS/cellular functionality. Cellular functionality can, for example, be 3G, 4G, or 5G functionality. The one or more communications controllers 330 can communicate information to the main controller 310. Any of the one or more communications controllers 330 can include internal memory or can utilize memory 350. Any of the one or more communications controllers 330 can be a low-power processor.

The control system 300 can store data, such as GPS data, therapy data, device data, and event data. This data can be stored, for example, in memory 350. This data can include patient data collected by one or more sensors. The control system 300 can track and log therapy and other operational data. Such data can be stored, for example, in the memory 350.

Using the connectivity provided by the one or more communications controllers 330, the control system 300 can upload any of the data stored, maintained, or tracked by the control system 300 to a remote computing device, such as the device 334. The control system 300 can also download various operational data, such as therapy selection and parameters, firmware and software patches and upgrades, and the like (for example, via the connection to the device 334). The one or more additional processors 380, such as processor for controlling one or more user interfaces (such as, one or more displays), can be utilized. In some cases, any of the illustrated or described components of the control system 300 can be omitted depending on an embodiment of a wound monitoring or treatment system in which the control system 300 is used.

Any of the negative pressure wound therapy devices described herein can include one or more features disclosed in U.S. Patent No. 9,737,649 or U.S. Patent Publication No. 2017/0216501.

### Sensor-Integrated Wound Dressing with Smart Disposable

A wound dressing that incorporates a number of electronic components, including one or more sensors, can be utilized in order to monitor characteristics of a wound. Collecting and analyzing data from a wound can provide useful insights towards determining whether a wound is on a healing trajectory, selecting proper therapy, determining whether the wound has healed, or the like. Any of the sensor-integrated wound dressings disclosed herein can be configured as a smart disposable.

In some implementations, a number of sensor technologies can be used in wound dressings or one or more components forming part of an overall wound dressing apparatus. For example, as illustrated in FIGS. 4A-4C, one or more sensors can be incorporated onto or into a substrate (such substrate can be referred to as "sensor integrated substrate"). The substrate illustrated as having a square shape, but it will be appreciated that the substrate may have other shapes such as rectangular, circular, oval, etc. In some cases, a substrate supporting one or more sensors can be provided as an individual material layer that is placed directly or indirectly over or in a wound. The sensor integrated substrate can be part of a larger wound dressing apparatus. In some cases, the sensor integrated substrate is part of a single unit dressing. Additionally or alternatively, the sensor integrated substrate can be placed directly or indirectly over or in the wound and then covered by a secondary wound dressing, which can include one or more of gauze, foam or other wound packing material, a superabsorbent layer, a drape, a fully integrated dressing like the Pico or Allevyn Life dressing manufactured by Smith & Nephew, or the like.

The sensor integrated substrate can be placed in contact with a wound and can allow fluid to pass through the substrate while causing little to no damage to the tissue in the wound. The substrate can be flexible, elastic, extensible, or stretchable or substantially flexible, elastic, extensible, or stretchable in order to conform to or cover the wound. For example, the substrate can be made from a stretchable or substantially stretchable material, such as one or more of polyurethane, thermoplastic polyurethane (TPU), silicone, polycarbonate, polyethylene, polyimide, polyamide, polyester, polyethelene tetraphthalate (PET), polybutalene tetreaphthalate (PBT), polyethylene naphthalate (PEN), polyetherimide (PEI), along with various fluropolymers (FEP) and copolymers, or another suitable material. In some cases, the substrate can include one or more flexible circuit boards, which can be formed of flexible polymers, including polyamide, polyimide (PI), polyester, polyethylene naphthalate (PEN), polyetherimide (PEI), along with various fluropolymers (FEP) and copolymers, or the like. One or more sensors can be incorporated into a two-layer flexible circuit board. In some scenarios, the one or more circuit boards can be a multi-layer flexible circuit board.

In some cases, the sensor integrated substrate can incorporate adhesive, such as a wound contact layer as described herein, that adheres to wet or dry tissue. In some cases, one or more sensors, which can be positioned one or more flexible circuit boards, can be incorporated into any layer of the wound dressing. For example, a wound contact layer can have cutouts or slits that allow for one or more sensors to protrude out of the lower surface of the wound contact layer and contact the wound directly. In some situations, one or more sensors can be incorporated into or encapsulated within other components of a wound dressing, such as an absorbent layer.

As shown in FIG. 4A, a sensor integrated substrate 500A can support a plurality of electronic components and a plurality of electronic connections interconnecting at least some of the components. The electronic components can be one or more of any electronic components described herein, such as a sensor, amplifier, capacitor, resistor, inductor, controller, processor, or the like. The electronic connections can electrically connect one or more of the electronic components. The electronic connections can be can be tracks printed on the substrate, such as using copper, conductive ink (such as silver ink, graphite ink, etc.), or the like. At least some of the electronic connections can be flexible or stretchable or substantially flexible or stretchable.

The plurality of electronic components can include one or more impedance or conductivity sensors 510, which can be arranged in an outer 4x4 grid and an inner 4x4 grid as illustrated in FIGS. 4A-4C. Sensors 510 are illustrated as pads configured to measure impedance or conductivity of tissue across any pair of the pads. Two (or more) excitation pads 515 can be arranged as illustrated to provide the excitation signal across the pads, which is conducted by the tissue and responsive to which impedance or conductance of the tissue can be measured across the pads 510. Electrical components, such as one or more amplifiers 520, can be used to measure impedance or conductance of the tissue. Impedance or conductance measurements can be used to identify living and dead tissue, monitor progress of healing, or the like. The arrangement of the pads 510 in the inner and outer grids can be used to measure the impedance or conductance of the wound, perimeter of the wound, or tissue or areas surrounding the wound. Additional approaches to measuring impedance or conductivity are described in International Patent Publication No. WO2020/187643, titled "Systems and Methods for Measuring Tissue Impedance".

The substrate 500A can be configured as a smart disposable and store status data. Impedance or conductance of one or more components of the substrate 500A, such as one or more of the impedance sensors 510, can be measured and stored in a memory of the substrate 500A as part of the status data. This can be performed during manufacturing or calibration of the substrate 500A. In use, the impedance or conductance stored as part of the status data can be used to accurately measure impedance or conductance of the wound or tissue. For instance, the impedance or conductance provided by the status data can be factored out (or otherwise disregarded) when determining the impedance or conductance of the wound or tissue.

The plurality of electronic components can include one or more temperature sensors 530 configured to measure temperature of the wound or surrounding tissue. For example, nine temperature sensors arranged around the perimeter of the substrate 500A. One or more temperature sensors can include one or more thermocouples or thermistors. One or more temperature sensors can be calibrated and the data obtained from the one or more sensors can be processed to provide information about the wound environment. In some cases, an ambient sensor measuring ambient air temperature can also be used to assist in eliminating problems associated with environment temperature shifts.

The plurality of electronic components can include one or more optical sensors 550. One or more optical sensors 550 can be configured to measure wound appearance or image the wound. In some cases, a light source or illumination source that emits light and a light sensor or detector that detects light reflected by the wound are used as one or more optical sensors. The light source can be a light emitting diode (LED), such as one or more of white LED, red, green, blue (RGB) LED, ultraviolet (UV) LED, or the like. The light sensor can be one or more of an RGB sensor configured to detect color, infrared (IR) color sensor, UV sensor, or the like. In some cases, both the light source and detector would be pressed up against the skin, such that light would penetrate into the tissue and take on the spectral features of the tissue itself. In some scenarios, one or more optical sensor can include an imaging device, such as a charge-coupled device (CCD), CMOS image sensor, or the like.

In some cases, ultra bright LEDs, an RGB sensor, and polyester optical filters can be used as components of the one or more optical sensors to measure through tissue color differentiation. For example, because surface color can be measured from reflected light, a color can be measured from light which has passed through the tissue first for a given geometry. This can include color sensing from diffuse scattered light, from an LED in contact with the skin, or the like. In some cases, an LED can be used with a proximal RGB sensor to detect the light which has diffused through the tissue. The optical sensors can image with diffuse internal light or surface reflected light.

Status data can include calibration data (such as, thresholds) for any the sensors supported by the substrate 500A. For example, sensitivity, color, or power for optical sensors can be included in the status data. As another example, impedance or conductance for the impedance sensors can be included in the status data. As yet another example, temperature thresholds for the temperatures sensors can be included in the status data. As yet another example, absolute pressure or humidity thresholds can be included in the status data. Such status data can be used to compensate for any variability between different substrates 500A.

Calibration data can be used to obtain accurate measurement of the wound or tissue in use. As described herein, status data can include an indication of the substrate 500 having been used on a patient. Responsive to such indication, a notification can be provided to the healthcare provider. This can facilitate ensuring that the substrate 500A is not used with a different patient (for instance, without having been cleaned and sterilized).

Status data can include data related to the capabilities of the substrate 500A. For instance, a particular substrate may only include temperature sensors. This data can be used to adjust one or more of collection of data or provision of therapy. Verification of a data collection or therapy prescription can be performed. For instance, suppose that collection of wound impedance is part of a prescription, but the status data of the substrate indicates that the substrate does not include impedance sensors. In such case, an indication (such as, alarm) can be generated. Such verification can be performed for any of the smart disposables described herein.

As described herein, status data can include an indication that end of life of the substrate 500A has been reached. Status data can facilitate ensuring and enforcing that the substrate 500A (or any other smart disposable described herein) is being used only for a single patient. For example, when a smart disposable is connected to a therapy or monitoring device (such as, the pump assembly 160 or a control module), the device can store its real time clock in the memory of the smart disposable. Any future parameter stored in the memory of the smart disposable can include a timestamp with real time clock. If there is a mismatch in the timestamps (such as, the real time clocks are not indicative of a passage of a particular duration of time), a determination that there is an attempt to use the smart disposable with a different therapy or monitoring device (and likely for a different patient) can be made. Such attempted use on a different patient can be prevented. Timestamps can be written periodically. A time stamp can be written responsive to a disconnection of the smart disposable from the therapy or monitoring device. Timestamps can be used to determine that the end of life of the smart disposable has been reached.

One or more of the plurality of electronic components can be controlled by a control module. The control module can receive and process one or more measurements obtained by the one or more sensors. An external control module can be connected to at least some of the plurality of electronic components via a connector 540. In some cases, the connector 540 can be positioned at the end of a conductive track portion as illustrated in FIG. 4B or attached to the conductive track portion at a position away from the end as illustrated in FIG. 4A or 4C (such as, attached to the top of the track portion with glue). The control module can include one or more controllers or microprocessors, memory, or the like. In some cases, one or more controllers can be positioned on the substrate, and the connector 540 is not used. In some cases, data and commands can be communicated wirelessly, such as by a transceiver positioned on the substrate, and the connector 540 is not used.

In some cases, additional or alternative sensors can be positioned on the substrate, such as one or more pH sensors, pressure sensors, perfusion sensors, or the like.

In some cases, a substrate can be perforated as illustrated in FIGS. 4B-4C. A plurality of perforations 560 can be formed in the substrate 500B, allowing fluid to pass through the substrate. It may be advantageous to use a perforated substrate in conjunction with application of negative pressure wound therapy, during which reduced pressure is applied to the wound covered by a dressing and which causes removal of fluid (such as wound exudate) from the wound. Perforations 560 can be formed around a plurality of electronic components and connections as illustrated in FIGS. 4B-4C. Perforations 560 can be formed as slits or holes. In some cases, perforations 560 can be small enough to help prevent tissue ingrowth while allowing fluid to pass through the substrate.

In some cases, any of the wound dressings or wound dressing components described herein can be part of a kit that also includes a negative pressure wound therapy device. One or more components of the kit, such as the sensor integrated substrate, secondary dressing, or the negative pressure wound therapy device can be sterile.

Any of the embodiments disclosed herein can be used with any of the embodiments described in International Patent Publication No. WO2017/195038, titled "SENSOR ENABLED WOUND MONITORING AND THERAPY APPARATUS," International Patent Publication No. WO2018/189265, titled "COMPONENT STRESS RELIEF FOR SENSOR ENABLED NEGATIVE PRESSURE WOUND THERAPY DRESSINGS," International Patent Publication No. WO2020/157103, titled "SENSOR INTEGRATED DRESSINGS AND SYSTEMS," International Patent Application No. PCT/EP2018/069886, titled "SKEWING PADS FOR IMPEDANCE MEASUREMENT," International Patent Application No. PCT/EP2018/075815, titled "SENSOR POSITIONING AND OPTICAL SENSING FOR SENSOR ENABLED WOUND THERAPY DRESSINGS AND SYSTEMS," and International Patent Application No. PCT/EP2018/069883, titled "BIOCOMPATIBLE ENCAPSULATION AND COMPONENT STRESS RELIEF FOR SENSOR ENABLED NEGATIVE PRESSURE WOUND THERAPY DRESSINGS".

### Wound Debridement with Smart Disposable

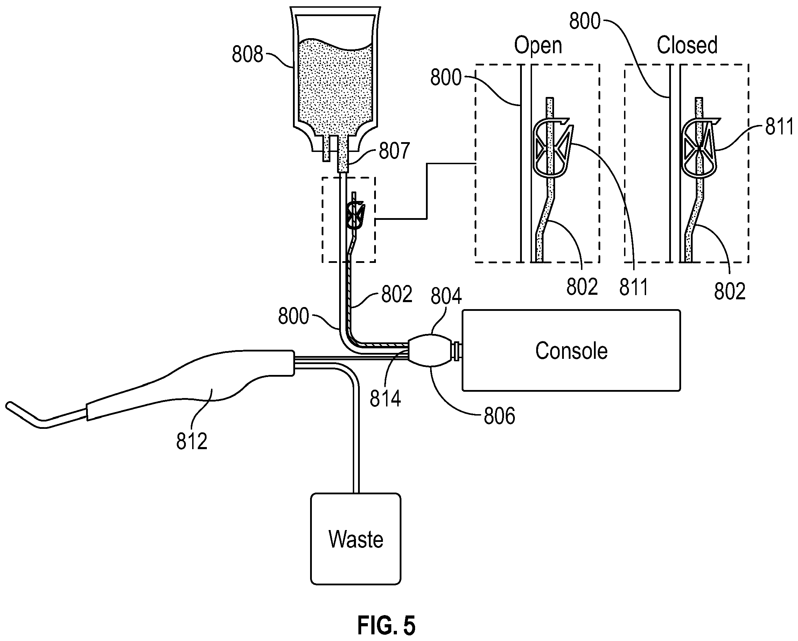

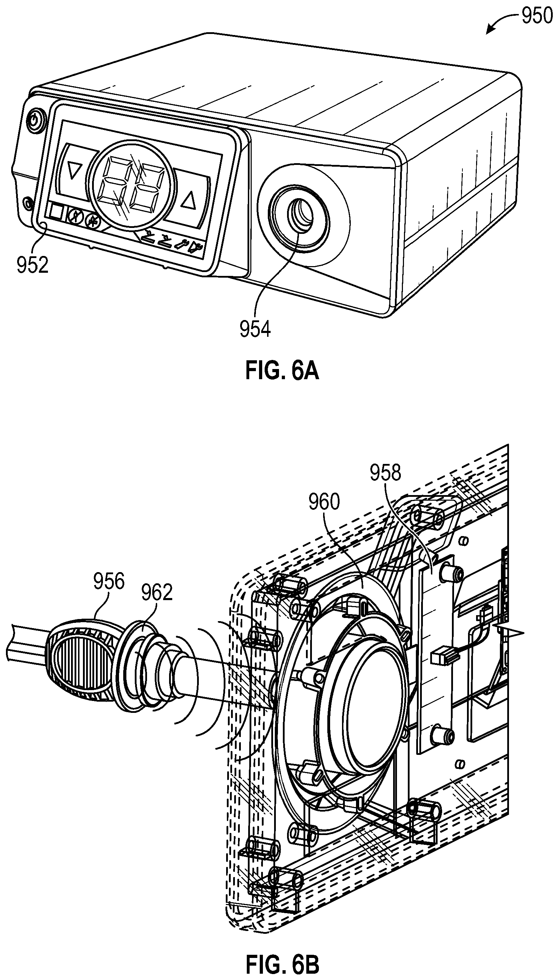

A wound debridement system can be used to promote wound healing. A debridement system, such as Smith & Nephew Versajet Hydrosurgery system, can utilize a high pressure water or other liquid for debridement. In some cases, a debridement system can use a high pressure saline water jet for debridement and wound bed preparation. Referring to Figure 5, a liquid jet debridement and wound bed preparation system can include two saline tubes 800, 802 extending from an inlet 804 of a pump handle 806. The pump can be a piston pump. A handpiece (or hand piece or handset) 812 can extend from an inlet 814 of the pump handle 806. The handpiece 812 can be configured to treat a wound with liquid pressure from a liquid jet of a fluid. For example, a pressurized liquid jet (or stream) can be emitted from the handpiece 812. Saline tube 800 can terminate in a spike 807 that connects to a saline bag 808. Saline tube 802 can be open to atmosphere or include a spike that connects to a second saline bag. In the system of Figure 5, air can be expelled from the open tube 802 with a clamp 811 in an open position when there is an airlock in the system. Referring to Figures 6A-6B, a console 950 of a liquid jet debridement and wound bed preparation system includes a display 952 (such as, an LCD screen) for displaying device state information, for example, power level, procedure running time, out-patient/operating room mode, and service reminders. The console 950 can include an interface 954 for receiving the pump 956 (which can be a piston pump), and a reader 958 and antenna 960, which can identify a tag 962 installed in a handpiece (such as, the handpiece 812). The identification can be performed using RFID or near-field (such as, NFC), in which case the tag 962 can be an RFID or NFC tag. As described herein, identification can be performed using infrared (such as, IrDA), vibration, or the like

The handpiece 812 can be configured as a smart disposable and store status data. Such handpiece 812 can facilitate system level activation and deactivation when connected to the console 950. The status data can include the exact pressure of the liquid jet of the handpiece 812. Such pressure can be used for calibration to provide a more precise pressure during use or to ensure patient safety. For instance, in the event that the status data indicates that the pressure of the handpiece is incompatible with the console 950, debridement can be disabled. This can prevent use of the liquid jet debridement with an improper pressure. As described herein, status data can include an indication of the handpiece 812 having been used on a patient. Responsive to such indication, the console 950 can disable debridement. This can facilitate ensuring that the handpiece 812 is not used for providing debridement to a different patient (for instance, without having been cleaned and sterilized). As described herein, end of life of the handpiece 812 can be monitored and detected.

Console can store as part of status data one or more of system failures with the handpiece, priming problems by measuring the time prior to proper procedure, duration of setting up, temperature during procedure, location, hand vibration level (which can be relevant safety of the user), procedure duration, failure(s) or intermittence(s), pressure decay, confirmation of pressure settings for a type of handpiece, noise acoustic value, flow, exhaust flow, misuse data, or the like.

In some cases, it may be important to ensure that the handpiece 812 is sterile when it is being used to treat a wound. The handpiece 812 can be positioned in a sterile package and thus remain sterile while packaged. However, when the handpiece 812 is taken out of the packaging, it can lose sterility after being exposed to environment outside of packaging (such as, atmosphere) for a period (or duration) of time following opening of the packaging. For instance, the period of time can be about 15 minutes (or less or more) at room temperature. As a smart disposable, the handpiece 812 can implement one or more mechanisms that monitor the period of time and provide a loss of sterility indication. One such mechanism can be a chemical-based trigger. For example, the handpiece 812 can include a reactive element or material that changes its state when exposed to air (as a result of one or more of oxidation or being exposed to humidity). In some cases, oxidation be preferrable as the oxygen content of a room typically does not vary as much as humidity or temperature.

The reactive material can be conductive, such as aluminum or iron. When the reactive material is exposed to air, it can lose its conductivity as a result of being oxidized (or being exposed to humidity). Responsive to the expiration of the period of time, the reactive material can become non-conductive (for instance, conductive iron can become non-conductive iron rust). The reactive material can be part of an electronic circuit that changes its state (such as, becomes an open circuit) as a result of the transition of the material from being conductive to becoming non-conductive. In some cases, oxidation (or humidity) can cause a mechanical break in a conductive pathway. For instance, a conductive wire, membrane, or the like can become broken. Such change of the state of the electronic circuit can be detected by the console 950. As a result, connection with of a non-sterile handpiece 812 (such as, open circuit) can be detected by the console, treatment can be disabled, and an optional indication can be provided to the user.

In some implementations, another such mechanism can be an enzymatic-based trigger. For example, an electronic circuit can include one or more photodiodes coated (or otherwise coupled) with a material that changes one or more properties (such as, transparency, color, or the like) responsive to being exposed to the environment. The material can change the one or more properties as a result of being cured when exposed to the environment. For example, the material can be silicone. Responsive to the expiration of a period of time (which may the same or different as the period of time in described in the preceding paragraph), the material can change the one or more properties that would cause the photodiode to detect (or stop detecting) light, which would lead to the change of the state of the electronic circuit. The material can be a color or polarizing filter for the one or more diodes. As a result, connection with of a non-sterile handpiece 812 (such as, open or closed circuit) can be detected by the console, treatment can be disabled, and an optional indication can be provided to the user.

While detection of loss of sterility is described in connection with the handpiece 812, approaches disclosed herein can be applied to any sensor-integrated substrates (such as, 500A or 500B), canisters, or other smart disposables described herein.

### Structure of Smart Disposable

Figure 7 illustrates a schematic of the smart disposable 1000. The smart disposable 1000 can include a processing circuitry 1002 (such as, one or more controllers), a transceiver 1004 for wired or wireless communications, one or more memories 1006, and a power source 1008. The memory 1006 can be non-volatile or persistent memory, such as EPROM or flash memory. Status data can be stored in the memory 1006. The transceiver can facilitate wireless communications using NFC, RFID, Bluetooth, WiFi, or the like. The communication between the transceiver 1004 and an external computing device (such as, a medical or diagnostic device including any such devices described herein) may be bidirectional. The control circuity 1002 may be connected to the memory 1006 and the transceiver 1004 and configured to store data in the memory 1006 and cause the transceiver 1004 to provide status data to the external computing device. The external computing device may be a wound therapy or treatment device or another computing device (such as, a phone, tablet, PC, or the like).

The power source 1008 is configured to supply power to the control circuity 1002, the transceiver 1004, and the memory 1006. The power source 1008 can include one or more capacitors or a battery (such as, a coin cell battery). The power source 1008 can be small since a large capacity power source may not be needed because the communication with the smart disposable would be short and infrequent. In some cases, the smart disposable 1000 can be powered by an external power source and the power source 1008 may be omitted. For example, external power can be provided via NFC or RFID (such as, in case the smart disposable 1000 is a passive tag).

The smart disposable 1000 can include a housing. For instance, in case of a canister (such as, the canister 162), the housing can store fluid aspirated from the wound. One or more of the processing circuitry 1002, the transceiver 1004, the one or more memories 1006, or the power source 1008 can be partially or fully enclosed by or positioned within the housing.

The external computing device can store, modify, and retrieve the status data. As described herein, status data can include, but is not limited to, instructions on how to recycle the smart disposable 1000, disassembly instructions, instructions for use, indication of past use of the smart disposable, regulatory information, or legal information. Recycling instructions can facilitate reduction of waste. For example, recycling instructions can be provided automatically (or on demand) responsive to the determination that the smart disposable 1000 has reached end of life. The transfer of information to the computing device can supplement or replace any traditional labeling (such as, text labels, bar code labels, QR code labels, etc.). This is advantageous as a traditional label can be damaged or destroyed. The external computing device can communicate at least part of the status data to a user. For instance, instructions can be displayed.

The smart disposable 1000 can facilitate writing status data to the one or more memories 1006. Status data can be communicated (such as, via a wireless or wired communication protocol) by an external computing device and stored in the one or more memories.

As an example, during manufacture, data can be written to the one or more memories 1006. For a canister, for instance, the data can include a coded value representing one or more of the manufacturing date of the canister and lot code. Device identification data (such as, a serial number) can be included in the data. The manufacturing data can be set with a permanent write lock after being written. When attached to a negative pressure wound therapy device, such manufacturing data can be read from and compared to the current date. A check can be performed to ensure that the canister is not past its indicated shelf life. If the shelf life of the canister has been exceeded, the negative pressure wound therapy device can generate an indication, which can include one or more of alerting the user to the out-of-date canister or refusing to operate with an out-of-date canister. Manufacturing data (or any other data described herein) may be read not just by the negative pressure wound therapy device, but by another external computing device that implements the communication protocol.

In some cases, identification of the smart disposable 1000 can be represented as impedance of capacitance (such as, resistor or capacitor) in addition to or instead of data stored in the one or more memories 1006.

Continuing with the canister example, during provision of therapy, the negative pressure wound therapy device can write data (for instance, in the format of coded values) indicative of therapy performance. Such data can include one or more of negative pressure set point(s), other therapy settings, alarm(s) or alert(s), fluid flow rate(s) during use (can be indicative of flow rate over duration of time, such as an hour), leak rate(s) during use (can be indicate of leak rate over duration of time, such as an hour), self-test result(s) (which can include a subset of last self-tests, such as last one, last two, or last three), motion data, or the like. One or more parameters for alarm(s) or alert(s) can be stored into the one or more memories 1006, such as one or more parameters for canister full detection, leakage detection, or the like. This can facilitate seamless provision of therapy with canisters of different sizes, volumes, or the like. Self-test results can include leak rate, flow rate, power requirements for flow, or the like. Motion data can include data related to the smart disposable or the device, such as detection of any sudden accelerations relative to an acceleration threshold (which can be indicative of fall or another impact), inversions, vibrations, or the like. For a handpiece (such as, the handpiece 812), written data can include motor current, speed data, pressure data, or the like. Data written to the smart disposable can facilitate determination of misuse, therapy or treatment failures, or the like.

Also, the negative pressure wound therapy device can write data indicating whether the canister was full or removed before being full. Such data can be written with a write lock as it may be important for determining whether reconnecting the canister with the negative pressure wound therapy device to continue therapy would be allowed. For instance, reconnecting the canister to continue therapy may not be allowed with a canister that has been detected to be full.

Further, data indicating that the canister is full can be written responsive to detecting that the canister has filled up for an appropriate percentage of a particular time period. This can prevent false positives. Such data can be written with a write lock.

Additionally, the negative pressure wound therapy device can write data indicative of loss of sterility (such as, "biohazard") responsive to initiation of therapy. This data can be used to prevent usage of a non-sterile canister for a different patient.

Moreover, the negative pressure wound therapy device can identify write-locked areas of the one or more memories 1006. For instance, the device may attempt to write a random code to one or more memory areas (or, in some cases, to each memory area) and read back the value. This can be performed responsive to the canister being connected to the device.

In some implementations, the smart disposable 1000 can utilize one or more antennas for wireless communication. To achieve redundancy, there can be multiple conductive paths to a single antenna (such as, from the transceiver 1004) or multiple antennas (and multiple transceivers 1004). Multiple antennas can utilize different frequencies (or frequency bands) to avoid or minimize interference. For example, as described herein, NFC protocol can be used for wireless communication. Transmission can be accomplished at 13.56 MHz frequency and EPC Gen2 protocol frequency 865-868 MHz (or 902-928 MHz). Communication with such smart disposable 1000 can be accomplished using multiple readers (each supporting a particular frequency or frequency band), multi-frequency reader, or single reader with multiple communication circuitries can be used (which can rotate, oscillate, or the like).

In some cases, the smart disposable 1000 can be utilized to update firmware or software on the medical or diagnostic device with which the smart disposable 1000 is being used. For example, a firmware or software patch (or code) can be stored in the one or more memories 1006 of the smart disposable 1000. This can be performed during manufacturing. In the field, when the device connects to the smart disposable 1000, the device can retrieve and install the patch. The device can retrieve and install the patch responsive to reading information stored in the one or more memories 1006 indicative of a firmware (or software) version of the device needed to operate with the disposable 1000. If the current version of the firmware (or software) running on the device does not match the information indicative of the required firmware (or software) version, the device and retrieve and install the patch. As another example, the device would retrieve and install the patch responsive to a determination that the device's firmware (or software) version is earlier (such as, lower) than smart disposable's firmware (or software) version or smart disposable's version. The patch can include one or more of data related to firmware (or software) upgrade or calibration data for the smart disposable 1000 or the device (such as, negative pressure set point, alerts, last self-test value, or the like). Advantageously, firmware (or software) updates of the device can be performed efficiently even for devices that do not support connectivity to the Internet or another network to complete a remote update. The need to perform local updates of the device or the need to transport the device for an update can be avoided.

Data stored in the one or more memories 1006 of the smart disposable 1000 can be displayed (or otherwise communicated to the user) via one or more user interfaces of the device (such as, the display 172). For instance, a message related to the need to install a firmware (or software) patch can be displayed. Displayed data can include information related to new products or other marketing information.

Data stored in the one or memories 1006, which can include patient data, can be encrypted. For a canister, patient data can include canister full indication (which can be indicative of the patient not being healthy), time duration for canister to be replaced, time to alarm, configuration of the system (such as, provision of therapy to multiple wounds using Y-connect), parameters of exudate stored in the canister, or the like. For a handpiece (such as, the handpiece 812), such patient data can include pressure, time of treatment, or the like. Event log can be saved in the one or more memories 1006. A key may be needed to decrypt the encrypted data. Stored data can be used to determine compliance, post-treatment market surveillance, or the like.

A smart disposable can store status data that can be accessed by a computing device (which can be a therapy or monitoring device). As a result, the entire lifecycle (from manufacturing to disposal) of the smart disposable can be effectively and efficiently managed. Patient safety and integrity of medical procedures can be improved. Complaints (including regulatory complaints) can be resolved efficiently and compliance with therapy or monitoring prescription can be improved.

### Other Variations

Any one or more features of a particular example of a smart disposable can be used with any of the other examples of a smart disposable.

Although some embodiments describe negative pressure wound therapy, the systems, devices, and/or methods disclosed herein can be applied to other types of therapies usable standalone or in addition to TNP therapy. Systems, devices, and/or methods disclosed herein can be extended to any medical device, and in particular any wound treatment or wound care device. For example, systems, devices, and/or methods disclosed herein can be used with devices that provide one or more of ultrasound therapy, oxygen therapy, neurostimulation, microwave therapy, active agents, antibiotics, antimicrobials, debridement, or the like. Such devices can in addition provide TNP therapy. The systems and methods disclosed herein are not limited to medical devices and can be utilized by any electronic device.

Any of transmission of data described herein can be performed securely. For example, one or more of encryption, https protocol, secure VPN connection, error checking, confirmation of delivery, or the like can be utilized.

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), and/or all of the steps of any method or process so disclosed, can be combined in any combination, except combinations where at least some of such features and/or steps are mutually exclusive. The protection is not restricted to the details of any foregoing embodiments, but solely defined by the set of claims. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated and/or disclosed may differ from those shown in the figures. Depending on the embodiment, certain of the steps described above may be removed, others may be added. For example, the actual steps and/or order of steps taken in the disclosed processes may differ from those shown in the figure. Depending on the embodiment, certain of the steps described above may be removed, others may be added. For instance, the various components illustrated in the figures or described herein may be implemented as software and/or firmware on a processor, controller, ASIC, FPGA, and/or dedicated hardware. The software or firmware can include instructions stored in a non-transitory computer-readable memory. The instructions can be executed by a processor, controller, ASIC, FPGA, or dedicated hardware. Hardware components, such as controllers, processors, ASICs, FPGAs, and the like, can include logic circuitry. Furthermore, the features and attributes of the specific embodiments disclosed above may be combined in different ways to form additional embodiments, all of which fall within the scope of the present disclosure.

User interface screens illustrated and described herein can include additional and/or alternative components. These components can include menus, lists, buttons, text boxes, labels, radio buttons, scroll bars, sliders, checkboxes, combo boxes, status bars, dialog boxes, windows, and the like. User interface screens can include additional and/or alternative information. Components can be arranged, grouped, displayed in any suitable order.

Conditional language used herein, such as, among others, "can," "could", "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied. Additionally, the words "herein," "above," "below," and words of similar import, when used in this application, refer to this application as a whole and not to any particular portions of this application.

Conjunctive language, such as the phrase "at least one of X, Y and Z," unless specifically stated otherwise, is to be understood with the context as used in general to convey that an item, term, etc. may be either X, Y, or Z, or a combination thereof. Thus, such conjunctive language is not generally intended to imply that certain embodiments require at least one of X, at least one of Y and at least one of Z to each be present.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

Unless otherwise explicitly stated, articles such as "a" or "an" should generally be interpreted to include one or more described items. Accordingly, phrases such as "a device configured to" are intended to include one or more recited devices. Such one or more recited devices can also be collectively configured to carry out the stated recitations.

Although the present disclosure includes certain embodiments, examples and applications, it will be understood by those skilled in the art that the present disclosure extends beyond the specifically disclosed embodiments to other alternative embodiments and/or uses and obvious modifications and equivalents thereof, including embodiments which do not provide all of the features and advantages set forth herein. Accordingly, the scope of the present disclosure is not intended to be limited by the specific disclosures of preferred embodiments herein, and is defined by claims as presented herein.

## Claims

1. A canister for negative pressure wound therapy, the canister comprising:
a housing defining an interior volume for storing fluid aspirated from a wound during provision of negative pressure wound therapy; and
an electronic circuitry at least partially enclosed by the housing, the electronic circuitry comprising:
a memory configured to store data related to one or more parameters of the canister;
a transceiver configured to wirelessly communicate with a computing device; and
a processing circuitry connected to the memory and transceiver, the processing circuitry configured to store the data in the memory and cause the transceiver to provide at least some of the data stored in the memory responsive to a request from the computing device.

2. The canister of claim 1, wherein the memory is further configured to store code for updating firmware or software of the computing device, and wherein the computing device comprises a negative pressure wound therapy device configured to removably support the housing.

3. The canister of claim 2, wherein the memory is further configured to store an indication for the negative pressure wound therapy device to determine that firmware or software should be updated with the code.

4. The canister of claim 3, wherein the indication comprises a version of firmware of software stored in the memory and configured to be executed by the processing circuitry.

5. The canister of any one of the preceding claims, wherein transceiver is configured to communicate with the computing device using near field communication (NFC), and wherein the computing device comprises a negative pressure wound therapy device configured to removably support the housing.

6. The canister of any one of the preceding claims, wherein the data comprises canister capacity stored in the memory prior to the canister being used for negative pressure wound therapy.

7. The canister of any one of the preceding claims, wherein the data comprises a canister orientation or canister fill status determined during provision of negative pressure wound therapy and stored in the memory during provision of negative pressure wound therapy.

8. The canister of claim 7, wherein the canister orientation comprises a flipped orientation determined during provision of negative pressure wound therapy, and wherein storing the flipped orientation in the memory prevents the canister from being used for further provision of negative pressure wound therapy.

9. The canister of any of the preceding claims, wherein the electronic circuitry further comprises a power source configured to supply power to at least one of the memory, the transceiver, or the processing circuitry.

10. The canister of claim 9, wherein the power source comprises one or more of a capacitor or a battery.

11. The canister of any one of the preceding claims, wherein the data comprises a manufacturing date stored as read only data, wherein the manufacturing date facilitates a determination that shelf life of the canister has not been exceeded.

12. The canister of any one of the preceding claims, wherein the data comprises an indication that the canister is full, the indication being stored responsive to a determination that a level of fluid aspirated from the wound has reached a threshold level over a duration of time.

13. A kit comprising a negative pressure wound therapy device and a canister as defined in any one of claims 1 to 12.

## Patentansprüche

1. Kanister für Unterdruck-Wundtherapie, wobei der Kanister umfasst:
ein Gehäuse, welches ein Innenvolumen zum Aufbewahren von Fluid definiert, das während der Bereitstellung einer Unterdruck-Wundtherapie aus einer Wunde abgesaugt wird; und
elektronische Schaltungen, die wenigstens teilweise von dem Gehäuse umschlossen sind, wobei die elektronischen Schaltungen dies umfassen:
einen Speicher, der dazu ausgelegt ist, Daten in Bezug auf einen oder mehrere Parameter des Kanisters zu speichern;
einen Sendeempfänger, der dazu ausgelegt ist, drahtlos mit einer Rechenvorrichtung zu kommunizieren; und
Verarbeitungsschaltungen, die mit dem Speicher und dem Sendeempfänger verbunden sind, wobei die Verarbeitungsschaltungen dazu ausgelegt sind, die Daten in dem Speicher zu speichern und den Sendeempfänger zu veranlassen, wenigstens einige der in dem Speicher gespeicherten Daten in Reaktion auf eine Anforderung von der Rechenvorrichtung bereitzustellen.

2. Kanister nach Anspruch 1, wobei der Speicher ferner dazu ausgelegt ist, Code zum Aktualisieren von Firmware oder Software der Rechenvorrichtung zu speichern, und wobei die Rechenvorrichtung eine Unterdruck-Wundtherapievorrichtung umfasst, die dazu ausgelegt ist, das Gehäuse abnehmbar zu unterstützen.

3. Kanister nach Anspruch 2, wobei der Speicher ferner dazu ausgelegt ist, eine Angabe für die Unterdruck-Wundtherapievorrichtung zu speichern, um zu bestimmen, dass Firmware oder Software mit dem Code aktualisiert werden sollten.

4. Kanister nach Anspruch 3, wobei die Angabe eine Version von Firmware oder Software umfasst, die in dem Speicher gespeichert ist und dazu ausgelegt ist, durch die Verarbeitungsschaltungen ausgeführt zu werden.

5. Kanister nach einem der vorstehenden Ansprüche, wobei der Sendeempfänger dazu ausgelegt ist, mit der Rechenvorrichtung unter Verwendung einer Nahfeldkommunikation (Near Field Communication, NFC) zu kommunizieren, und wobei die Rechenvorrichtung eine Unterdruck-Wundtherapievorrichtung umfasst, die dazu ausgelegt ist, das Gehäuse abnehmbar zu unterstützen.

6. Kanister nach einem der vorstehenden Ansprüche, wobei die Daten eine Kanisterkapazität umfassen, die in dem Speicher gespeichert ist, bevor der Kanister zur Unterdruck-Wundtherapie verwendet wird.

7. Kanister nach einem der vorstehenden Ansprüche, wobei die Daten eine Kanisterausrichtung oder einen Kanisterfüllstatus umfassen, die während der Bereitstellung einer Unterdruck-Wundtherapie bestimmt werden und die während der Bereitstellung einer Unterdruck-Wundtherapie in dem Speicher gespeichert werden.

8. Kanister nach Anspruch 7, wobei die Kanisterausrichtung eine umgedrehte Ausrichtung umfasst, die während der Bereitstellung einer Unterdruck-Wundtherapie bestimmt wird, und wobei das Speichern der umgedrehten Ausrichtung in dem Speicher verhindert, dass der Kanister zur weiteren Bereitstellung einer Unterdruck-Wundtherapie verwendet wird.

9. Kanister nach einem der vorstehenden Ansprüche, wobei die elektronischen Schaltungen ferner eine Stromquelle umfassen, die dazu ausgelegt ist, Strom für wenigstens eines von dem Speicher, dem Sendeempfänger oder den Verarbeitungsschaltungen zu liefern.

10. Kanister nach Anspruch 9, wobei die Stromquelle eines oder mehrere von einem Kondensator oder einer Batterie umfasst.

11. Kanister nach einem der vorstehenden Ansprüche, wobei die Daten ein Fertigungsdatum umfassen, das als schreibgeschütztes Datum gespeichert ist, wobei es das Fertigungsdatum ermöglicht zu bestimmen, dass die Haltbarkeit des Kanisters nicht überschritten worden ist.

12. Kanister nach einem der vorstehenden Ansprüche, wobei die Daten eine Angabe umfassen, dass der Kanister voll ist, wobei die Angabe in Reaktion auf eine Bestimmung gespeichert wird, dass der Pegel eines aus der Wunde abgesaugten Fluids über einen Zeitraum hinweg ein Schwellwertniveau erreicht hat.

13. Kit, umfassend eine Unterdruck-Wundtherapievorrichtung und einen Kanister wie in einem der Ansprüche 1 bis 12 definiert.

## Revendications

1. Cartouche pour la thérapie de plaies par pression négative, la cartouche comprenant :
un boîtier définissant un volume intérieur destiné à stocker un fluide aspiré à partir d'une plaie pendant la mise en œuvre d'une thérapie de plaies par pression négative ; et
un ensemble de circuit électronique au moins partiellement enfermé dans le boîtier, l'ensemble de circuit électronique comprenant :
une mémoire configurée pour stocker des données liées à un ou plusieurs paramètres de la cartouche ;
un émetteur-récepteur conçu pour communiquer sans fil avec un dispositif informatique ; et
un circuit de traitement connecté à la mémoire et à l'émetteur-récepteur, le circuit de traitement étant configuré pour stocker les données dans la mémoire et amener l'émetteur-récepteur à fournir au moins certaines des données stockées dans la mémoire en réponse à une demande du dispositif informatique.

2. Cartouche selon la revendication 1, la mémoire étant en outre configurée pour stocker un code pour mettre à jour un micrologiciel ou un logiciel du dispositif informatique, et le dispositif informatique comprenant un dispositif de thérapie de plaies par pression négative configuré pour supporter de manière amovible le boîtier.

3. Cartouche selon la revendication 2, la mémoire étant en outre configurée pour stocker une indication pour le dispositif de thérapie de plaies par pression négative afin de déterminer que le micrologiciel ou le logiciel doit être mis à jour avec le code.

4. Cartouche selon la revendication 3, l'indication comprenant une version du micrologiciel d'un logiciel stocké dans la mémoire et configuré pour être exécuté par les circuits de traitement.

5. Cartouche selon l'une quelconque des revendications précédentes, l'émetteur-récepteur étant configuré pour communiquer avec le dispositif informatique en utilisant une communication en champ proche (NFC), et le dispositif informatique comprenant un dispositif de thérapie de plaies par pression négative configuré pour supporter de manière amovible le boîtier.

6. Cartouche selon l'une quelconque des revendications précédentes, les données comprenant une capacité de cartouche stockée dans la mémoire avant que la cartouche soit utilisée pour une thérapie de plaies par pression négative.

7. Cartouche selon l'une quelconque des revendications précédentes, les données comprenant une orientation de cartouche ou un état de remplissage de cartouche déterminé pendant la fourniture d'une thérapie de plaies par pression négative et stocké dans la mémoire pendant la fourniture d'une thérapie de plaies par pression négative.

8. Cartouche selon la revendication 7, l'orientation de la cartouche comprenant une orientation retournée déterminée pendant la fourniture d'une thérapie de plaies par pression négative, et le stockage de l'orientation retournée dans la mémoire empêchant que la cartouche soit utilisée pour fournir une thérapie de plaies par pression négative supplémentaire.

9. Cartouche selon l'une quelconque des revendications précédentes, l'ensemble de circuit électronique comprenant en outre une source d'alimentation configurée pour alimenter au moins l'un de la mémoire, de l'émetteur-récepteur ou du circuit de traitement.

10. Cartouche selon la revendication 9, la source d'alimentation comprenant un ou plusieurs d'un condensateur ou d'une batterie.

11. Cartouche selon l'une quelconque des revendications précédentes, les données comprenant une date de fabrication stockée en tant que données en lecture seule, la date de fabrication facilitant une détermination que la durée de conservation de la cartouche n'a pas été dépassée.

12. Cartouche selon l'une quelconque des revendications précédentes, les données comprenant une indication que la cartouche est pleine, l'indication étant stockée en réponse à une détermination qu'un niveau de fluide aspiré à partir de la plaie a atteint un niveau de seuil au cours d'une durée de temps.

13. Kit comprenant un dispositif de thérapie de plaies par pression négative et une cartouche selon l'une quelconque des revendications 1 à 12.
